**Europäisches Patentamt**

**European Patent Office**

**Office européen des brevets**

(19)

(11) Veröffentlichungsnummer: **0 352 588 B1**

## (12) EUROPÄISCHE PATENTSCHRIFT

(45) Veröffentlichungstag der Patentschrift: **21.09.94**

(51) Int. Cl.⁵: **A61L 25/00**, A61L 27/00

(21) Anmeldenummer: **89113070.0**

(22) Anmeldetag: **17.07.89**

Die Akte enthält technische Angaben, die nach dem Eingang der Anmeldung eingereicht wurden und die nicht in dieser Patentschrift enthalten sind.

(54) **Verbesserte körperresorbierbare Knochenwachse (III).**

(30) Priorität: **25.07.88 DE 3825211**

(43) Veröffentlichungstag der Anmeldung:
**31.01.90 Patentblatt 90/05**

(45) Bekanntmachung des Hinweises auf die
Patenterteilung:
**21.09.94 Patentblatt 94/38**

(84) Benannte Vertragsstaaten:
**AT BE CH DE ES FR GB GR IT LI LU NL SE**

(56) Entgegenhaltungen:
**EP-A- 0 250 994**
**US-A- 4 443 430**
**US-A- 4 645 503**

(73) Patentinhaber: **Henkel Kommanditgesellschaft auf Aktien**

**D-40191 Düsseldorf (DE)**

(72) Erfinder: **Fues, Johann-Friedrich, Dr.**
**Herzogstrasse 15**
**D-4048 Grevenbroich (DE)**
Erfinder: **Ritter, Wolfgang, Dr.**
**Am Bandenfeld 74**
**D-5657 Haan (DE)**

(74) Vertreter: **von Kreisler, Alek, Dipl.-Chem. et al**
**Patentanwälte**
**von Kreisler-Selting-Werner**
**Postfach 10 22 41**
**D-50462 Köln (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 352 588 B1

## Beschreibung

Die EP-A2-0 250 994 beschreibt die Verwendung von fließfähigen bis festen oligomeren Estern der Milchsäure und/oder der Glykolsäure und ihren dermatologisch verträglichen Derivaten als resorbierbare Träger und/oder Filmbildner in Mitteln zur Abdeckung unverletzter und/oder verletzter Bereiche menschlicher oder tierischer Haut. Im einzelnen sieht die Lehre dieser Druckschrift vor, daß oligomere Träger und/oder Filmbildner mit einem Gehalt an freien Carboxylgruppen verwendet werden können, wobei solche freien Carboxylgruppen bei der Anwendung des Behandlungsmittels auf noch blutende frische Wunden zu einer erwünschten Blut-Koagulation führen können. In der Erfindungsbeschreibung dieser Druckschrift ist dann aber auch angegeben, daß die in den oligomeren Reaktionsprodukten der Milchsäure und/oder der Glykolsäure gegebenenfalls vorliegenden freien Carboxylgruppen in ihre Salze umgewandelt werden können. Hierzu werden pharmakologisch unbedenkliche Basen verwendet.

Gegenstand der offengelegten deutschen Patentanmeldung P 32 29 540.5 (D 6652) sind resorbierbare Wachse zur mechanischen Blutstillung an körpereigenem Hartgewebe, insbesondere Knochen, die sich dadurch auszeichnen, daß sie aus bei Körpertemperatur zähviskosen bis festen wachsartigen Polyester-Oligomeren von niederen Hydroxycarbonsäuren bestehen. Aufgrund ihrer Struktur sind diese Wachse durch körpereigene Stoffwechselmechanismen abbaubar, wobei die Geschwindigkeit des Abbaus in an sich bekannter Weise einstellbar ist. Oligomere der Glykolsäure werden vom körpereigenen Stoffwechsel schneller abgebaut als solche der Milchsäure. Die Abbaugeschwindigkeit ist damit beispielsweise durch Mischveresterung der beiden genannten Oxycarbonsäuren regulierbar. Die bevorzugten Wachse weisen mittlere Molekulargewichte im Bereich von etwa 200 bis 1 500 und insbesondere im Bereich von etwa 300 bis 1 000 auf.

Zur Regelung des mittleren Molekulargewichts dieser Polyester-Oligomeren schlägt die genannte Offenlegungsschrift vor, monofunktionelle und/oder difunktionelle Alkohole oder Carbonsäuren bzw. Carbonsäure-Anhydride und/oder primäre bzw. sekundäre Monoamine mitzuverwenden. In an sich bekannter Weise kann dann durch Wahl geeigneter Mischungsverhältnisse an Oxycarbonsäure und zusätzlicher monofunktioneller bzw. difunktioneller Komponente ein sich letztlich einstellendes mittleres Molekulargewicht vorherbestimmt werden. Die dabei anfallenden Reaktionsprodukte sind bekanntlich bezüglich ihres Oligomerisationsgrades nicht einheitlich, sie enthalten auch noch gewisse Anteile an eingesetzten Ausgangskomponenten.

Gegenstand der älteren deutschen Patentanmeldung P 37 16 302.7 (D 7890) ist eine weiterführende Optimierung solcher resorbierbarer Wachse zur mechanischen Blutstillung an körpereigenem Hartgewebe. Beschrieben ist in diesem älteren Schutzrecht, daß besonders körper- und insbesondere gewebeverträgliche Wachse dann erhalten werden, wenn - unter Wahrung der allgemeinen Gesetzmäßigkeiten aus der genannten veröffentlichten deutschen Schutzrechtsanmeldung - zur Einstellung des mittleren Molekulargewichts ein ganz bestimmter dreifunktioneller Alkohol, nämlich Glycerin, eingesetzt wird. Die Kombination von Glycerin mit Oligoestern der Milchsäure und/ oder der Glykolsäure führt zu abbaubaren wachsartigen Komponenten der genannten Art, die sich bei der Implantation in lebendes Körpergewebe durch eine besonders gut ausgeprägte Körperverträglichkeit auszeichnen.

Das genannte ältere Schutzrecht der Anmelderin schildert dann weiterhin, daß unerwünschte Gewebsschädigungen besonders sicher vermieden werden können, wenn durch die Herstellung und eine anschließende Reinigung des abbaubaren Wachses sichergestellt wird, daß dessen Gehalt an nichtreagierten Carboxylgruppen zumindest stark abgesenkt oder besser noch praktisch vollständig beseitigt wird. Solche freien Carboxylgruppen können auch noch dann im Reaktionsgemisch vorliegen, wenn auf die Mitverwendung von Molekulargewichts-regelnden Dicarbonsäuren im Sinne des erwähnten vorveröffentlichten Vorschlages der Anmelderin verzichtet wird. Die statistische Molekulargewichtsverteilung, die sich unmittelbar aus der Herstellung der Wachse ergibt, läßt noch immer einen gewissen Anteil an freien Carboxylgruppen im oligomeren Reaktionsgemisch zurück, die zumindest vorwiegend als freie, monomere Hydroxycarbonsäuren vorliegen. Das gilt auch dann, wenn an sich der gewünschte mittlere Zahlenwert für das Molekulargewicht eingestellt ist.

Nach der älteren Anmeldung wird in einer besonders bevorzugten Ausführungsform der dort geschilderten Erfindung das wachsartige Material von seinem Gehalt an nicht umgesetzten Ausgangskomponenten wenigestens weitgehend befreit. Hierbei soll insbesondere der Gehalt an nicht reagierten Hydroxycarbonsäuren auf Restgehalte unterhalb 0,5 Gew.-% und vorzugsweise auf Restgehalte unter etwa 0,2 Gew.-% abgesenkt werden. In der Regel werden nach dem älteren Vorschlag die Restgehalte an nicht reagierten Hydroxycarbonsäuren in den zum Einsatz kommenden Wachsen bei oder unterhalb von 0,1 Gew.-% liegen.

Die Entfernung der nicht umgesetzten Reaktanten soll dabei bevorzugt mittels eines Umfällungsverfahrens erfolgen, wobei die erwünschten Oligomerfraktionen aus einer zuvor hergestellten Lösung bzw.

2

Aufschlämmung ausgefällt werden, die nicht umgesetzten Komponenten und/oder Reaktionsprodukte niederen Molekulargewichts jedoch in Lösung verbleiben.

Die Lehre der vorliegenden Erfindung geht von der Aufgabe aus, körperresorbierbare Knochenwachse der geschilderten Art in mehrfacher Weise weiterhin zu verbessern. Hierbei stehen insbesondere die folgenden Erwägungen im Vordergrund:

Auch erfindungsgemäß soll eine erhöhte Gewebeverträglichkeit des in den menschlichen oder tierischen Körper implantierten Wachses erzielt werden, wobei insbesondere der Gehalt an freien Carboxylgruppen im Reaktionsprodukt abgesenkt und am besten beseitigt sein soll. Die Erfindung legt aber darüberhinaus weiterhin Wert auf die praktische Handhabbarkeit der Wachse, um die Arbeit des Operateurs zu erleichtern. Hier ist der folgende Sachverhalt zu berücksichtigen: Oligomerprodukte der betroffenen Art und insbesondere Oligoester der Milchsäure zeichnen sich häufig durch eine derart ausgeprägte Zähviskosität aus, daß die praktische Handhabung des Wachses im Operationsbetrieb Schwierigkeiten bereiten kann. Das Fadenziehen der aus physiologischen Überlegungen besonders erwünschten milchsäurereichen Oligoester behindert die rasche und störungsfreie Handhabung bei der Materialentnahme und beim Knochenverschluß. Entsprechende Schwierigkeiten treten bei oligomeren Estern der Glykolsäure nicht oder in untergeordnetem Maße auf. Aus physiologischen Erwägungen kann aber gerade der Einsatz entsprechender Wachse auf überwiegender Milchsäurebasis wünschenswert sein. Der Abbau der Glykolsäure im körpereigenen Reaktionsgeschehen läuft bekanntlich über die Oxalsäure, nicht aber der der Milchsäure. Ist die Verhinderung oder die Einschränkung zusätzlicher Oxalsäurebildung im Körpergeschehen angezeigt, dann werden für operative Eingriffe der hier betroffenen Art Wachse auf überwiegender Basis der Milchsäure besonders wichtig. Ihrer vereinfachten praktischen Handhabung kommt damit beträchtliche Bedeutung zu.

Die Lehre der Erfindung zur Lösung dieser vielseitigen Aufgabenstellung geht von der Erkenntnis aus, daß in einfacher Weise hohe Gewebeverträglichkeit, verbesserte Handhabung auch sonst nur schwierig zu verarbeitender zähviskoser Wachse bei gleichzeitig hohen Gehalten an Milchsäure als chemische Grundeinheit des Polyestermoleküls durch Einführung von körperverträglichen Carbonsäuresalzgruppen in das Reaktionsgemisch erreicht werden kann. Anstelle der Entfernung der nicht reagierten bzw. niedermolekularen Anteile mit freiem Carboxylgruppengehalt aus dem oligomeren Reaktionsprodukt sieht die Erfindung die Neutralisation dieser Säuregruppen mit geeigneten Basen vor. Es werden dabei zusätzliche Anteile körperverträglicher Salze, z. B. an entsprechenden Salzen der monomeren Hydroxyconbonsäuren, dem wachsartigen Reaktionsprodukt zugesetzt; hierdurch wird insbesondere die Handhabung sonst zähviskoser, fadenziehender Reaktionsprodukte substantiell erleichtert.

Gegenstand der Erfindung ist dementsprechend die Verwendung von

(a) Oligomer-Wachsen der Glykolsäure und/oder Milchsäure sowie deren Derivaten mit 1 - und/oder mehrfunktionellen Alkoholen und/oder entsprechenden Carbonsäuren

in homogener Abmischung mit

(b) körperverträglichen Salzen organischer und/oder anorganischer Säuren, die durch Abreaktion der im Oligomer-Wachs vorliegenden freien Rest-Carboxylgruppen gebildet und gewünschtenfalls darüberhinaus als zugesetzte Salze homogen verteilt in das Wachs eingearbeitet worden sind, dabei in Mengen von nicht mehr als 30 Gew.-% - bezogen auf Oligomeren-Wachs - vorliegen und Teilchengrößen bis 250 $\mu$m aufweisen,

Zur Herstellung körperresorbierbarer zähviskoser bis fester Knochenwachse zur mechanischen Blutstillung an körpereigenem Hartgewebe.

Geeignete Basen zur Bildung der Carbonsäuresalzgruppen sind vor allem Alkalien, Erdalkalien und/oder Aluminium; besondere Bedeutung kommt dabei entsprechen Salzgruppen des Natriums, des Caliums und/oder des Magnesiums zu. Durch den Einbau solcher Salzgruppen wird nicht nur Einfluß auf die Gewebeverträglichkeit und die Verarbeitbarkeit der Knochenwachse genommen, es kann auf diese Weise der Knochenneubildung in situ wertvolles biologisches Material zur Verfügung gestellt werden.

Zur Umwandlung zähplastischer und besonders fadenziehender Produkte - wie angegeben insbesondere bei Wachsen auf Basis von Milchsäure als polyesterbildendem Molekül - werden in einer bevorzugten Ausführungsform der Erfindung zur Verbesserung der Verarbeitbarkeit Salze der Glykolsäure und/oder der Milchsäure zugesetzt. Auch hier sind als salzbildende Kationen die zuvor genannten Basen bevorzugt. Die Lehre der Erfindung sieht vor, den Gehalt des Gesamtgemisches an diesen zugesetzten Salzen monomerer Oxycarbonsäuren auf 30 Gew.-% zu beschränken. Im allgemeinen ist es zweckmäßig, den Gehalt an solchen Salzen auf nicht mehr als 20 Gew.-% und insbesondere auf nicht mehr als 10 Gew.-% zu beschränken. Hierbei sind die Gewichtsprozent-Angaben jeweils bezogen auf das Oligomerenwachs.

Neben oder anstelle solcher Salze organischer Carbonsäuren und insbesondere der genannten Salze der Glykolsäure und/oder der Milchsäure können zur Verbesserung der Verarbeitbarkeit den Oligomerkomponenten körperverträgliche Salze anorganischer Säuren zugesetzt sein. Es hat sich überraschenderweise gezeigt, daß beispielsweise ein hoch zähviskoses Oligomerprodukt auf Milchsäurebasis schon durch homogene Einmischung sehr beschränkter Mengen solcher anorganischer feinstteiliger Salze in ein deutlich besser handhabbares und verarbeitbares Mischprodukt umgewandelt werden kann. Dabei können schon mit Mengen unterhalb 10 Gew.-% - bezogen auf Oligomergewicht - des Salzzusatzes befriedigende Verarbeitungseigenschaften sichergestellt werden. Grundsätzlich ist allerdings auch hier die Zugabe größerer Mengen anorganischer Salze als zur Erfindung gehörig anzusehen. Die zuvor für die organischen Salze genannten Grenzwerte gelten sinngemäß.

Besonders geeignete anorganische Salzkomponenten gehen in ihrer chemischen Beschaffenheit auf keramische Werkstoffe zurück, wie sie in Literatur und Praxis heute für Knochenersatzwerkstoffe vorgeschlagen sind. Die wichtigste Klasse sind Calciumphosphat-Keramikwerkstoffe, die als körperresorbierbares Material ausgebildet sein können. Aber auch schwer resorbierbare Calciumphosphatkeramik in Pulverform fällt in den Rahmen der hier geschilderten Ausführungsform der Erfindung.

Vergleichsweise leicht körperresorbierbare Calciumphosphate sind das Tricalciumphosphat und seine Abkömmlinge, die im Austausch gegen Calcium- und/oder Phosphat-ionen andere Ionen, z. B. solche des Fluors, $CO_3$-Reste, Magnesium und dergleichen in das Kristallgitter aufgenommen enthalten. Ein solcher Partialersatz der Ursprungsbestandteile des Tricalciumphosphats führt nicht nur zu Veränderungen bzw. Störungen der Gitterstruktur, sondern auch zu abweichendem Löslichkeitsverhalten. So steigt beispielsweise die Löslichkeit mit dem Carbonatgehalt, während sie durch Fluoraufnahme sinkt.

Ein anderes erfindungsgemäß besonders geeignetes Zusatzmittel dieser Art ist der sogenannte Hydroxylapatit, der als Pentacalcium-Hydroxid-Triphosphat der Formel $Ca_5 OH(PO_4)_3$ anzusprechen ist. Ein pulverförmiger Hydroxylapatit kann in der primär anfallenden biologisch vergleichsweise leicht resorbierbaren Form eingesetzt werden. Geeignet sind aber auch gesinterte Keramiken dieser Art, die man z. B. aus gefälltem Calciumphosphatpulver bei erhöhten Temperaturen erhalten kann. Material dieser Art zeigt eine erhöhte Widerstandsfähigkeit in biologischem Milieu. Zur einschlägigen Literatur zum Einsatz sogenannter bioaktiver Werkstoffe auf Calciumphosphatbasis im lebenden Körper wird beispielsweise auf die folgenden Veröffentlichungen verwiesen: "Resorbierbare keramische Werkstoffe für den Knochenersatz", Biomedizinische Technik, Band 20, Mai 1975, Seite 115 ff.; "Neuere Werkstoffe in der medizinischen Technik", Zeitschrift "Chemie-Ingenieur-Technik",Heft 8, 1975, Seite 327 bis 333 oder DE-PS 22 42 867. Der Einsatz von partikulärem Calciumsulfat in der Außenfläche eines inplantierbarem Kunststoffkörpers und letztlich damit im lebenden Organismus ist beispielsweise beschrieben in der US-PS 3 919 773. Im weiteren Sinne fallen unter die hier diskutierten anorganischen Salzzusätze schließlich auch gepulverte bzw. gemahlene Partikel aus heterologem Knochenmaterial, beispielsweise entsprechende Zusatzstoffe, die aus einem dem sogenannten Kieler Knochenspan entsprechenden Material hergestellt worden sind. Nach den Angaben der US-PS 3 918 100 kann durch Einsatz eines solchen Mahlgutes aus Knochenpartikeln das Wachstum des natürlichen Knochengewebes stimuliert werden, wobei das eingebrachte Mahlgut im Laufe der Zeit durch neugebildete Knochen ersetzt wird.

Bei der Einarbeitung von in Wachs wenigstens weitgehend unlöslichen Salzen bzw. entsprechenden Knochenfeststoffen der zuvor beschriebenen Art ist es erfindungsgemäß bevorzugt absolute Teilchengrößen der individuellen Feststoffpartikel unterhalb etwa 100 μm einzuhalten. Durch die Wahl dieser unlöslichen Feststoffteilchen in so feinpulverisierter Form wird nicht nur die homogene Einmischung sichergestellt, es hat sich auch gezeigt, daß in der praktischen Anwendung Entmischungsvorgänge nicht zu befürchten sind, wie sie bei größeren Partikeln auftreten können. Die durchschnittliche Teilchengröße dieser erfindungsgemäß bevorzugten Pulver-Zusatzstoffe liegt unter 100 μm und insbesondere im Bereich von etwa 1 bis 50 μm.

Bevorzugt Zu verwendende Wachse sind unter Mitverwendung von Glycerin bzw. Glycerinpartialestern zur Regelung des mittleren Molekulargewichts der Oligomerenfraktion hergestellt worden, so wie es für die besonders körper- und gewebeverträglichen Wachse in der älteren Anmeldung P 37 16 302.7 (D 7890) beschrieben ist. Anstelle der dort geschilderten nachfolgenden Abtrennung nicht umgesetzter Reaktionsprodukte mit freier Carboxylgruppe wird erfindungsgemäß aber die Beseitigung der freien Carbonsäuregruppierungen durch Salzbildung mit den geschilderten Basen vorgenommen. Neben oder anstelle des Glycerins hat sich der Einsatz von Glycerinpartialestern mit insbesondere Monocarbonsäuren als Teilveresterungskomponente bewährt. Mono- bzw. Diglyceride dieser Art regeln aufgrund ihres Gehalts an freien Hydroxylgruppen in an sich bekannter Weise das mittlere Molekulargewicht, gleichzeitig wird über die am Partialglycerid vorliegende Säure eine weitere Komponente in das Knochenwachs eingeführt, die insbesondere wirksam zur weiteren Einflußnahme auf die Verarbeitungseigenschaften der Wachse eingesetzt werden kann. Physiologisch verträgliche Säuren für die Bildung der Glycerinpartialester sind insbesondere längerkettige Carbonsäuren, beispielsweise solche des C-Bereichs von $C_8$ bis $C_{20}$, insbesondere $C_{12}$ bis $C_{18}$.

Wird etwa durch Mitverwendung von Talgfettsäure-Partialglyceriden die Einstellung des gewünschten mittleren Molekulargewichts der polyesterbildenden Komponenten bewirkt, so fallen Reaktionsprodukte an, die schon aus sich heraus eine erleichterte Verarbeitung mit dem Spatel erlauben.

In einer besonderen Ausführungsform der Erfindung wird von der folgenden Erscheinung Gebrauch gemacht: An sich zähviskose und hoch fadenziehende Oligoester auf Milchsäurebasis können in ihren Verarbeitungseigenschaften schon dadurch substantiell verbessert werden, daß man sie mit vorzugsweise geringeren Mengen an Oligomeren der Glykolsäure mischt. Die stärker zur Kristallisation neigenden Glykolsäureoligomeren wirken sich dabei schon dann merklich aus, wenn sie in Mengen von etwa 5 bis 15 Gew. -% mit den Milchsäureoligomeren abgemischt werden. In dieser Ausführungsform der Erfindung enthält ein solches Gemisch aus einerseits Oligomeren der Milchsäure und andererseits Oligomeren der Glykolsäure bevorzugt wenigstens 35 Gew.-% und zweckmäßig wenigstens 50 Gew.-% an Milchsäureoligomeren. Besonders geeignet können in solchen Gemischen 75 bis 95 Gew.-% an Milchsäureoligomeren - und jeweils zum Rest des Oligomerengemisches Glykolsäureoligomere - vorliegen. Alle Gewichtsprozent-Angaben beziehen sich in diesem Zusammenhang auf das jeweils eingesetzte Oligomerengemisch.

In allen geschilderten Ausführungsformen - insbesondere also auch bei der Mitverwendung von monomeren Salzen der Milchsäure und/oder Glykolsäure - kann es erfindungsgemäß bevorzugt sein, daß im fertigen Stoffgemisch wenigstens 35 Mol-% und bevorzugt wenigstens 50 Mol-% an Milchsäureresten vorliegen - jeweils bezogen auf die Summe von Milchsäure- und Glykolsäureresten im oligomeren und gegebenenfalls monomeren Zustand. In besonders wichtigen Ausführungsformen überwiegt das Milchsäuremolekül im Stoffgemisch, so daß beispielsweise wenigstens 60 Mol-%, vorzugsweise wenigstens 75 Mol-% oder gar wenigstens 80 bis 90 Mol-% der Oxycarbonsäurereste auf die Milchsäure zurückgehen.

Im übrigen gelten weitgehend die Angaben der genannten DE-OS 32 29 540.5 zur Beschaffenheit und den Parametern der Wachse. So sind auch die erfindungsgemäßen Polyester-Oligomere durch ein mittleres Molekulargewicht im Bereich von etwa 200 bis 1 500 und vorzugsweise im Bereich von etwa 300 bis 1 000 gekennzeichnet. Die resorbierbaren Wachse sind bei Körpertemperatur pastös- bis weich-verstreichbare Materialien, die auch durch kurzzeitiges Erwärmen auf Temperaturen bis etwa 100 °C oder vorzugsweise bis etwa 60 °C in einen noch besser verstreichbaren Zustand gebracht werden können. In dieser Form eignen sie sich insbesondere zur mechanischen Blutstillung durch an sich bekannten Auftrag auf körpereigenes Gewebe, beispielsweise am verletzten oder sonstwie eröffneten Knochen.

Ausgangsmaterial für die Hestellung der Oligomeren können die monomeren Hydroxycarbonsäuren der genannten Definition sein. Man kann aber auch die leicht handhabbaren Dimerisationsprodukte, d. h. das Lactid und/oder das Glycolid einsetzen. Die Milchsäure bzw. das Milchsäuredimere kann als optisch aktive Komponente beispielsweise L-Lactit oder auch als ein Gemisch der optisch aktiven Verbindungen als D,L-Lactid Verwendung finden.

Bevorzugte Wachse im Sinne der Erfindung werden erhalten, wenn man jeweils auf ein Mol Glycerin etwa 9 bis 10 Mol Glykolsäure bzw. etwa 10 bis 12 Mol Milchsäure - jeweils gerechet als monomere Oxycarbonsäure - einsetzt. Einzelheiten zur Durchführung der Oligomerisationsreaktion finden sich in der zitierten Veröffentlichung gemäß DE-OS 32 29 540.

Die erfindungsgemäß vorgesehene Salzbildung an freien Carboxylgruppen im Reaktionsgemisch kann mit den entsprechenden freien Basen und/oder mit geeigneten Salzen dieser Basen vorgenommen werden. Geeignete Salze sind beispielsweise insbesondere die Carbonate und/oder Bicarbonate.

Zusätzliche Reinigungsvorgänge, beispielsweise durch Umkristallisation des Reaktionsgemisches, können vorgenommen werden, sind jedoch im allgemeinen nicht erforderlich.

In einer weiteren Ausführungsform der Erfindung werden neben den erfindungsgemäß definierten Wachsen auf Basis von Oligomeren der Glykolsäure und/oder der Milchsäure bzw. deren Derivaten mit 1 - und/oder mehrfunktionellen Alkoholen und/oder entsprechenden Carbonsäuren andere, in der Praxis bereits bekannte Knochenwachse mitverwendet. In Betracht kommen hier insbesondere Abmischungen mit Erdalkalisalzen höherer Fettsäuren, insbesondere des C-Zahlbereichs von 12 bis 18. Entsprechende Calciumsalze, hier insbesondere Calciumpalmitat und/oder Calciumstearat, können als solche Mischungskomponenten bevorzugt sein. Die Menge dieser mitverwendeten Mischungskomponenten beträgt vorzugsweise nicht mehr als 50 Gew.-% - bezogen auf das Gesamtwachs - und insbesondere nicht mehr als 35 Gew.-%. Durch Vermischen der Bestandteile in der gemeinsamen Schmelze können homogene Mischungszustände leicht eingestellt werden.

Auch für diese Ausführungsformen gilt die erfindungsgemäße Regel der Mitverwendung physiologisch verträglicher Salze anorganischen und/oder organischen Ursprungs der zuvor geschilderten Art. Ihre Mitverwendung ermöglicht hier ebenso wie in den zuvor geschilderten Ausführungsformen der Erfindung zusätzlich auch die vorteilhafte Einarbeitung von weiteren Wachstumsfaktoren für eine beschleunigte Knochenregeneration und Knochenneubildung. Verwiesen wird in diesem Zusammenhang beispielsweise auf die Veröffentlichung von W. Krüger et al. in "Dtsch. zahnärztl. Z." 43, 709 bis 712 (1988). Beschrieben ist hier die im Tierexperiment gefundene induzierte Knochenregeneration durch Einsatz von Hydroxylapatit und daran adsorbiertem wachstumförderndem mitogenen Protein.

Beispiele

In den nachfolgenden Beispielen werden vier Oligomerwachse auf Basis Glykolsäure/Glycerin (A, B) bzw. auf Basis Lactid/Glycerin (C, D) als Grundmaterialien für die jeweiligen Versuchsreihen eingsetzt.
Es werden dabei die folgenden Untersuchungen durchgeführt:
- Neutralisation der freien Säuregruppen mit äquimolaren Mengen an Calciumcarbonat;
- Abmischung der Harztypen einerseits mit Calciumglykolat und andererseits mit Calciumlactat;
- Zumischung von Hydroxylapatit zu den Harzen;
- Zumischung beschränkter Mengen des neutralisierten Harzes B auf Glykolsäurebasis zu dem neutralisierten Harz C auf Milchsäurebasis.

Die Neutralisation der freien Säuregruppen und die Einmischungsvorgänge werden jeweils in der Schmelze bei 100 °C unter Rühren vorgenommen. Dazu wurde von den unter 2. beschriebenen Knochenwachsen durch Titration der Gehalt an freier Säure (Glykolsäure/Milchsäure) bestimmt. Die freie Säure wurde dann durch Zugabe der äquimolaren Menge $CaCO_3$ und intensiver Durchmischung neutralisiert.

Die eingesetzten Materialien, die Mischprodukte und die zur Beurteilung eingesetzten Meßmethoden sind im folgenden beschrieben.

1. Meßmethoden und verwendete Zuschlagstoffe

a. Aussehen: Visuelle Beurteilung
b. Viskositätsmessung:
Bestimmung der Kegelplatten-Viskosität mit dem nachfolgenden Gerät: EIC VISCO-Plot der Fa. Epprecht Instruments und Controls AG/Schweiz mit REL Kegel-Platte; die Messungen wurden durchgeführt mit dem Meßkörper D bei 20 Upm und 100 °C (Einstellung 1).
Fadenziehen:
Die zu prüfenden Materialmuster werden in eine Aluminiumschale gegossen und nach 24stündigem Erkalten wie folgt überprüft:
c. Bestimmung der Fadenziehneigung
Ein Metallspatel (Breite 9 mm) wird senkrecht in die Wachsschicht gedrückt und nach einer Eindringtiefe von 3 mm langsam nach oben gezogen. Gemessen wird die Fadenlänge in cm bis zum Abriß.
d. Hydroxylapatit Pulver
penta-Cacliumhydroxy-triphosphat
$Ca_5(PO_4)_3(OH)$
Fa. Merck/Darmstadt
Teilchengröße 2 bis 100 $\mu$m
e. Ca-Glycolat/Ca-Lactat
Die DL-Milchsäure (Racemat) bzw. Glycolsäure wird in der dreifachen Menge Wasser gelöst, im Wasserbad auf ~ 70 °C erwärmt und mit der äquimolaren Menge $CaCO_3$ durch portionsweise Zugabe neutralisiert. Anschließend wird am Rotavapor bei 80 bis 90 °C bei ~ 10 mbar das Wasser vollständig abgezogen.
Das erhaltene, grobkörnige Material wurde im Mixer (Fa. Braun) pulverfrei zerkleinert.

2. Herstellung und Beschreibung der resorbierbaren Ausgangswachse

a. Wachse, hergestellt aus Glycolsäure/Glycerin
Allgemeine Vorschrift zur Herstellung der Umsetzungsprodukte von Glycolsäure mit Glycerin
In einem Dreihalskolben mit Rührer und Destillationsbrücke werden Glycolsäure und Glycerin vorgelegt. Unter vollständiger Inertisierung mit Stickstoff wird auf 150 °C aufgeheizt und die Reaktion innerhalb von 3 bis 5 h so lange weitergeführt, bis sich kein Reaktionswasser mehr abspaltet. Dann wird bei 150 °C vorsichtig auf 10 Torr evakuiert. Nach weiteren 2 h bei diesen Reaktionsbedingungen wird auf 100 °C abgekühlt, das Vakuum aufgehoben, wie zuvor beschrieben neutralisiert und das Produkt noch heiß abgefüllt.
Die Zusammensetzung der Ansätze und die Oligomereigenschaften sind der Tabelle 1 zu entnehmen.

## Tabelle 1

### Oligohydroxycarbonsäuren aus Glycolsäure und Glycerin

| Beispiel | Edukte | | Ausbeute Reaktionswasser % | Konsistenz bei Raumtemperatur | Viskosität/100°C MK-D bei 20 UpM | Fadenziehen (in cm bis zum Abriß) |
|---|---|---|---|---|---|---|
| | Glycolsäure mol | Glycerin mol | | | | |
| A | 8 | 1 | 100 | trübe, hochviskos | 5000 mPas | 2 |
| | | nach Neutralisation | | trübe, hochviskos | 5000 mPas | 7 |
| B | 9 | 1 | 99,1 | trübe, hochviskos | 10000 mPas | 2 |
| | | nach Neutralisation | | trübe, hochviskos | 10000 mPas | 7 |

b. Wachse, hergestellt aus Lactid und Glycerin

Allgemeine Vorschrift zur Herstellung der Umsetzungsprodukte von Lactid mit Glycerin

Lactid (L(-)-Lactid N, der Firma Böhringer Ingelheim) und Glycerin wurden in einer üblichen Laborapparatur unter Stickstoff und unter Rühren innerhalb von einer Stunde auf 195 °C erwärmt. Man ließ dann 3 Stunden bei 195 °C reagieren und füllte nach Neutralisation heiß ab. Als Katalysator war eine Sn-II-Chlorid-Lösung in Ether zugesetzt (7 ml einer Lösung von 2,5 g SnCl₂ in 1000 ml Ether bei der Umsetzung von 3 mol Lactid mit einem mol Glycerin).
Die Zusammensetzung und die Oligomereigenschaften sind in Tabelle 2 angegeben.

Tabelle 2

Oligohydroxycarbonsäuren aus Glycerin und Lactid

| Beispiel | Edukte | | Konsistenz bei Raumtemperatur | Viskosität/100°C MK-D bei 20 UpM | Fadenziehen (in cm bis zum Abriß) |
| --- | --- | --- | --- | --- | --- |
| | Glycerin mol | Lactid mol | | | |
| C | 1 | 5 | klar, viskos | 8000 mPas | ca. 60 |
| | | nach Neutra-lisation | trübe, hochviskos | 10000 mPas | 30 |
| D | 1 | 6 | klar, viskos | 10000 mPas | ca. 50 |
| | | nach Neutra-lisation | trübe, hochviskos | 10000 mPas | 30 |

3. Verfahren zur Befüllung der Knochenwachse und Eigenschaften der Mischungen

Die Schmelze der neutralisierten Knochenwachse wurde unter Rühren bei Temperaturen von 100 bis 120 °C mit folgenden Substraten befüllt:

a. Ca-Glycolat (2,5 - 20 %)
b. Ca-Lactat (2,5 - 20 %)
c. Hydroxylapatit (2,5 - 20 %)

Nach der Befüllung wurde für 15 Min. intensiv durchgemischt. Nach dem Erkalten (Lagerung für 24 Std. in einer Aluminiumschale) wurde die Konsistenz, die Fadenziehneigung und die Kugelplatten-Viskosität bei 100 °C beurteilt.

Die Ergebnisse sind in Tabellen 3 bis 12 aufgeführt.

Tabelle 3

| Knochenwachs | Zugabe von Ca-Glycolat/Gew.-% | Konsistenz bei Raumtemperatur | Viskosität/100 °C MK-D bei 20 UpM | Fadenziehen (in cm bis zum Abriß) |
|---|---|---|---|---|
| A | 2,5 | wachsartig | 25000 mPas | kein Fadenziehen |

Tabelle 4

| Knochenwachs | Zugabe von Hydroxylapatit | Konsistenz bei Raumtemperatur | Viskosität/100 °C MK-D bei 20 UpM | Fadenziehen (in cm bis zum Abriß) |
|---|---|---|---|---|
| A | 2,5 | wachsartig | 25000 mPas | kein Fadenziehen |
| | 5,0 | wachsartig | 41000 mPas | kein Fadenziehen |
| | 7,5 | wachsartig | 61000 mPas | kein Fadenziehen |

Tabelle 5

| Knochenwachs | Zugabe von Ca-Glycolat/Gew.-% | Konsistenz bei Raumtemperatur | Viskosität/100 °C MK-D bei 20 UpM | Fadenziehen (in cm bis zum Abriß) |
|---|---|---|---|---|
| B | 2,5 | wachsartig | 61000 mPas | größer 1 cm |
| | 5 | wachsartig | 66000 mPas | kein Fadenziehen |
| | 7,5 | wachsgriesartig teilkristallin | 71000 mPas | kein Fadenziehen |
| | 10 | wachsgriesartig teilkristallin | 76000 mPas | kein Fadenziehen |
| | 12,5 | wachsgriesartig | 92000 mPas | kein Fadenziehen |
| | 15 | wachsgriesartig | 97000 mPas | kein Fadenziehen |
| | 17,5 | wachsgriesartig trocken | 102000 mPas | kein Fadenziehen |
| | 20 | wachsgriesartig trocken | 112000 mPas | kein Fadenziehen |

Tabelle 6

| Knochenwachs | Zugabe von Ca-Lactat/ Gew.-% | Konsistenz bei Raumtemperatur | Viskosität/100°C MK-D bei 20 UpM | Fadenziehen (in cm bis zum Abriß) |
|---|---|---|---|---|
| B | 2,5 | trübe, hochviskos | 87000 mPas | ca. 1 |
| | 5 | trübe, hochviskos | 92000 mPas | ca. 1 |
| | 7,5 | wachsartig | 102000 mPas | |
| | 10 | wachsartig | 115000 mPas | |
| | 12,5 | wachsartig-griesartig | 122000 mPas | kein Faden-ziehen wachsartig |
| | 15 | wachsartig-griesartig | 125000 mPas | |
| | 17,5 | wachsartig-griesartig | 128000 mPas | |
| | 20 | wachsartig-griesartig | 133000 mPas | |

Tabelle 7

| Knochenwachs | Zugabe von Ca-Glycolat/Gew.-% | Konsistenz bei Raumtemperatur | Viskosität/100 °C MK-D bei 20 UpM | Fadenziehen (in cm bis zum Abriß) |
|---|---|---|---|---|
| C | 2,5 | trübe, hochviskos | 10000 mPas | 12 |
| | 5 | trübe, hochviskos | 10000 mPas | 10 |
| | 7,5 | trübe, hochviskos | 20000 mPas | 6 |
| | 10 | trübe, hochviskos | 30000 mPas | 5 |
| | 12,5 | trübe, teilkristallin | 41000 mPas | 4 |
| | 15 | trübe, teilkristallin | 51000 mPas | 4 |
| | 17,5 | trübe, wachsartig, teilkristallin | 66000 mPas | 3 |
| | 20 | trübe, wachsartig | 76000 mPas | 3 |

Tabelle 8

| Knochenwachs | Zugabe von Ca-Lactat/Gew.-% | Konsistenz bei Raumtemperatur | Viskosität/100°C MK-D bei 20 UpM | Fadenziehen (in cm bis zum Abriß) |
|---|---|---|---|---|
| C | 2,5 | trübe, hochviskos | 10000 mPas | 12 |
| | 5 | trübe, hochviskos | 15000 mPas | 8 |
| | 7,5 | trübe, hochviskos | 30000 mPas | 6 |
| | 10 | trübe, hochviskos | 40000 mPas | 5 |
| | 12,5 | trübe, teilkristallin | 61000 mPas | 3 |
| | 15 | trübe, teilkristallin | 71000 mPas | 2 |
| | 17,5 | trübe, teilkristallin | 92000 mPas | ca. 2 |
| | 20 | trübe, wachsartig | 107000 mPas | ca. 2 |

Tabelle 9

| Knochenwachs | Zugabe von Hydroxylapatit Pulver/Gew.-% | Konsistenz bei Raumtemperatur | Viskosität/100°C MK-D bei 20 UpM | Fadenziehen (in cm bis zum Abriß) |
|---|---|---|---|---|
| C | 5 | trübe, hochviskos | 15000 mPas | 7 |
| | 7,5 | trübe, hochviskos | 22000 mPas | 6 |
| | 10 | trübe, hochviskos | 39000 mPas | 5 |
| | 15 | trübe, hochviskos | 61000 mPas | 3 |

Tabelle 10

| Knochenwachs | Zugabe von Ca-Lactat/Gew.-% | Konsistenz bei Raumtemperatur | Viskosität/100°C MK-D bei 20 UpM | Fadenziehen (in cm bis zum Abriß) |
|---|---|---|---|---|
| D | 12,5 | trübe, teilkristallin | 67000 mPas | kein Fadenziehen |

Tabelle 11

| Knochenwachs | Zugabe von Hydroxylapatit Pulver/Gew.-% | Konsistenz bei Raumtemperatur | Viskosität/100°C MK-D bei 20 UpM | Fadenziehen (in cm bis zum Abriß) |
|---|---|---|---|---|
| D | 5 | trübe, hochviskos | 39000 mPas | 5 |
| | 7,5 | trübe, hochviskos | 61000 mPas | 3 |
| | 10 | trübe, hochviskos | 67000 mPas | 3 |
| | 15 | trübe, hochviskos | 78000 mPas | 1 |
| | 20 | hochviskoswachsartig | 90000 mPas | kein Fadenziehen |

In einer nachfolgenden Versuchsreihe wird das neutralisierte Knochenwachs C mit 5, 10 und 20 Gew.-% an neutralisiertem Knochenwachs B versetzt. Die an den jeweiligen Mischprodukten bestimmten Werte sind in der nachfolgenden Tabelle 12 zusammengefaßt.

Tabelle 12

| Zugabe von Knochenwachs B in Gew.-% | Konsistenz bei Raumtemperatur | Viskosität/100 °C MK-D bei 20 UpM | Fadenziehen (in cm bis zum Abriß) |
|---|---|---|---|
| 5 | trübe, hochviskos | 10000 mPas | 27 |
| 10 | hochviskos, teilkristallin | 20500 mPas | 20 |
| 20 | hochviskos, teilkristalliert | 41000 mPas | 17 |

**Patentansprüche**

1. Verwendung von

   (a) Oligomer-Wachsen der Glykolsäure und/oder Milchsäure sowie deren Derivaten mit 1 - und/oder mehrfunktionellen Alkoholen und/oder entsprechenden Carbonsäuren

   in homogener Abmischung mit

   (b) körperverträglichen Salzen organischer und/oder anorganischer Säuren, die durch Abreaktion der im Oligomer-Wachs vorliegenden freien Rest-Carboxylgruppen gebildet und gewünschtenfalls darüberhinaus als zugesetzte Salze homogen verteilt in das Wachs eingearbeitet worden sind, dabei in Mengen von nicht mehr als 30 Gew.-% - bezogen auf Oligomeren-Wachs - vorliegen und Teilchengrößen bis 250 $\mu$m

   aufweisen, Zur Herstellung, körperresorbierer zähviskoser bis fester Knochenwachse zur mechanischen Blutstillung an körpereigenem Hartgewebe.

2. Verwendung nach Anspruch 1, dadurch gekennzeichnet, daß Abmischungen verwendet werden, die Carbonsäuresalzgruppen von Alkali, Erdalkali und/oder Aluminium und dabei insbesondere solche Salzgruppen des Natriums, Calciums und/oder Magnesiums enthalten.

3. Verwendung nach Ansprüchen 1 und 2, dadurch gekennzeichnet, daß Abmischungen mit verbesserter Verarbeitbarkeit verwendet werden, die als Salze organischer Säuren solche der Glykolsäure und/oder der Milchsäure und/oder als Salze anorganischer Säuren körperverträgliche Keramikwerkstoffe, insbesondere auf Calciumphosphat-Basis, enthalten, wobei bevorzugt der Gehalt an diesen zugesetzten Salzen nicht mehr als 20 Gew.-% und insbesondere nicht mehr als 10 Gew.-% - jeweils bezogen auf Oligomeren-Wachs - ausmacht.

4. Verwendung nach Ansprüchen 1 bis 3, dadurch gekennzeichnet, daß Abmischungen verwendet werden, die als anorganischen Salzzusatz körperresorbierbare und/oder nicht-resorbierbare Calciumphosphate, insbesondere Hydroxylapatit und/oder Tricalciumphosphat, enthalten.

5. Verwendung nach Ansprüchen 1 bis 4, dadurch gekennzeichnet, daß Abmischungen verwendet werden, die im Oligomeren-Wachs weitgehend unlösliche Salze mit Teilchengrößen unterhalb 100 $\mu$m und insbesondere mit mittleren Teilchengrößen im Bereich von 1 bis 50 $\mu$m aufweisen.

6. Verwendung nach Ansprüchen 1 bis 5, dadurch gekennzeichnet, daß Abmischungen verwendet werden, deren Oligomerkomponenten durch Regelung des Molgewichts unter Mitverwendung von Glycerin und/oder Glycerinpartialestern mit insbesondere längerkettigen Fettsäuren und gewünschtenfalls nachfolgende Salzbildung am Restgehalt der im Reaktionsprodukt verbliebenen freien Carboxylgruppen hergestellt worden sind.

7. Verwendung nach Ansprüchen 1 bis 6, dadurch gekennzeichnet, daß Abmischungen verwendet werden, deren Gehalt an Milchsäureresten wenigstens 35 Mol-%, bevorzugt wenigstens 50 Mol-% - bezogen auf die Summe von Milchsäure- und Glykolsäureresten - beträgt.

8. Verwendung nach Ansprüchen 1 bis 7, dadurch gekennzeichnet, daß Abmischungen verwendet werden, die in der Oligomerkomponente Gemische von Milchsäureoligomeren und Glykolsäureoligomeren enthalten, wobei der Anteil an Milchsäureoligomeren wenigstens 35 Gew.-%, bevorzugt wenigstens 50 Gew.-% und insbesondere 75 bis 95 Gew.-% ausmacht, Gew.-% jeweils bezogen auf das Oligomerengemisch.

9. Verwendung nach Ansprüchen 1 bis 8, dadurch gekennzeichnet, daß Abmischungen eingesetzt werden, deren Oligomerkomponente ein mittleres Molekulargewicht im Bereich von etwa 200 bis 1500, vorzugsweise im Bereich von etwa 300 bis 1000 besitzt.

10. Verwendung nach Ansprüchen 1 bis 9, dadurch gekennzeichnet, daß Abmischungen verwendet werden, die bei Körpertemperatur pastös bis weich verstreichbar sind oder durch kurzzeitiges Erwärmen auf Temperaturen bis etwa 100 °C in einen pastösverstreichbaren Zustand gebracht werden können und dabei kein oder nur geringes Fadenziehen aufweisen.

**Claims**

1.  Use of
    (a) oligomer waxes of glycolic acid and/or lactic acid as well as of derivatives thereof with mono- and/or polyfunctional alcohols and/or corresponding carboxylic acids
    in a homogeneous mixture with
    (b) body-compatible salts of organic and/or inorganic acids, which salts have been formed by reaction of residual free carboxyl groups present in the oligomer wax and, if desired, moreover have been incorporated in the wax as added salts in a homogeneous distribution, said salts being present in amounts of not more than 30% by weight, relative to the oligomer wax, and having particles sizes of up to 250 $\mu$m,
    for the preparation of body-resorbable highly viscous to solid bone waxes for mechanical staunching of the blood on endogenous hard tissue.

2.  Use according to claim 1, characterized in that mixtures are used which contain carboxylic acid salt groups of alkali metal, alkaline earth metal and/or aluminium, more specifically salt groups of sodium, calcium and/or magnesium.

3.  Use according to claims 1 and 2, characterized in that mixtures having an improved processability are used which contain salts of glycolic acid and/or lactic acid as salts of organic acids and/or body-compatible ceramic materials, especially those based on calcium phosphate, as salts of inorganic acids, with the content of these added salts being preferably not more than 20% by weight, and especially not more than 10% by weight, each relative to the oligomer wax.

4.  Use according to claims 1 to 3, characterized in that mixtures are used which contain body-resorbable and/or non-resorbable calcium phosphates, especially hydroxylapatite and/or tricalcium phosphate, as the inorganic salt additive.

5.  Use according to claims 1 to 4, characterized in that mixtures are used which comprise salts that are largely insoluble in the oligomer wax and have particle sizes below 100 $\mu$m, and especially average particle sizes within the range of from 1 to 50 $\mu$m.

6.  Use according to claims 1 to 5, characterized in that mixtures are used the oligomer components of which have been produced by controlling the molecular weight by concomitant use of glycerol and/or partial glycerol esters with fatty acids, especially those having longer chains, and, if desired, subsequent salt formation with the residual content of the free carboxyl groups having remained in the reaction product.

7.  Use according to claims 1 to 6, characterized in that mixtures are used which contain lactic acid moieties in an amount of at least 35% by mole, and preferably at least 50% by mole, relative to the sum of lactic acid and glycolic acid moieties.

8.  Use according to claims 1 to 7, characterized in that mixtures are used which contain mixtures of lactic acid oligomers and glycolic acid oligomers in the oligomer component, the amount of lactic acid oligomers being at least 35% by weight, preferably at least 50% and especially from 75 to 95% by weight, % by weight each based on the oligomer mixture.

9.  Use according to claims 1 to 8, characterized in that mixtures are used the oligomer component of which has an average molecular weight within the range of from about 200 to 1500, preferably within

EP 0 352 588 B1

the range of from about 300 to 1000.

10. Use according to claims 1 to 9, characterized in that mixtures are used which are from pasty to soft-spreadable at body temperature or can be converted into a pasty-spreadable form by brief heating to a temperature of up to about 100 °C and exhibits no or just minor ropiness.

**Revendications**

1. Utilisation de :

a) cires d'oligomères de l'acide glycolique et/ou de l'acide lactique ainsi que leurs dérivés comprenant des alcools mono et/ou plurifonctionnels et/ou des acides carboxyliques correspondants, en mélange homogène avec

b) des sels compatibles pour le corps d'acides organiques et/ou minéraux, qui ont été formés par réaction des groupes carboxyles libres résiduels présents dans la cire d'oligomères et si on le désire en outre ont été insérés réparties de manière homogène comme sels ajoutés dans la cire, en outre ils sont présents en quantités non supérieures à 30 % en poids - rapporté à la cire d'oligomères et ont des particules de grandeur allant jusqu'à 250 $\mu$m,

pour la fabrication de cire d'os résorbable par le corps, de l'état visqueux à l'état solide, pour l'hémostase mécanique sur du tissu dur propre du corps.

2. Utilisation selon la revendication 1, caractérisée en ce que, on utilise des mélanges qui contiennent des groupes de sels à'acide carboxylique d'alcalis, d'alcalino-terreux et/ou d'aluminium en outre en particulier des groupes de sels du sodium, du calcium et/ou du magnésium.

3. Utilisation selon les revendications 1 et 2, caractérisée en ce que on utilise des mélanges avec une possibilité de traitement améliorée qui contiennent comme sels d'acides organiques ceux de l'acide glycolique et/ou de l'acide lactique et/ou comme sels d'acides minéraux des matières céramiques supportables par le corps, en particulier à base de phosphate de calcium, de préférence la teneur de ces sels ajoutés n'étant pas fixée à plus de 20 % en poids et en particulier à plus de 10 % en poids, rapporté respectivement à la cire d'oligomères.

4. Utilisation selon les revendications 1 à 3, caractérisée en ce que on utilise des mélanges, qui contiennent comme addition de sels minéraux des phosphates de calcium résorbables par le corps et/ou non résorbables, en particulier de l'hydroxylapatite et/ou du phosphate tricalcique.

5. Utilisation selon les revendications 1 à 4, caractérisée en ce que on utilise des mélanges qui comportent des sels largement insolubles avec des grandeurs de particules inférieures à 100 $\mu$m et en particulier avec des grandeurs de particules au niveau de 1 à 50 $\mu$m.

6. Utilisation selon les revendications 1 à 5, caractérisée en ce que on utilise des mélanges, dont des composants oligomères ont été produits avec réglage du poids moléculaire en utilisant du glycérol et/ou des esters partiels du glycérol avec des acides gras en particulier à chaîne plus longue et si on le souhaite avec formation successive de sel sur la teneur résiduelle des groupes carboxyles libres demeurés dans le produit réactionnel.

7. Utilisation selon les revendications 1 à 6, caractérisée en ce que on utilise des mélanges, dont la teneur en esters d'acide lactique atteint au moins 35 % molaire, de préférence au moins 50 % molaire, rapporté à la somme de l'acide lactique et des esters d'acide glycolique.

8. Utilisation selon les revendications 1 à 7, caractérisée en ce que on utilise des mélanges, qui contiennent dans le composant oligomère des mélanges d'oligomères d'acide lactique et d'oligomères d'acide glycolique, la proportion d'oligomères d'acide lactique se montant à au moins 35 % en poids, de préférence à au moins 50 % en poids et en particulier de 75 à 95 % en poids, pourcentage en poids respectivement rapporté au mélange d'oligomères.

9. Utilisation selon les revendications 1 à 8, caractérisé en ce que on utilise des mélanges, dont le composant oligomère possède un poids moléculaire moyen au ni-veau environ de 200 à 1500, de préférence au niveau environ de 300 à 1000.

14

10. Utilisation selon les revendications 1 à 9, caractérisée en ce que on utilise des mélanges qui à la température du corps sont pâteux à mous et susceptibles d'être étendus ou peuvent être amenés par un bref réchauffement à des températures jusqu'à environ 100°C dans un état pâteux susceptible d'être étendu et en outre présentent aucun ou seulement un état de filage faible.